# EUROPEAN PATENT APPLICATION

(11) **EP 4 091 565 A1**
(43) Date of publication of application: **23.11.2022**
(21) Application number: 22173662.2
(22) Date of filing: 17.05.2022
(51) Int. Cl.: A61B 18/14, A61B 18/00

(54) **A CATHETER WITH MULTIPLE PHYSICALLY SYMMETRICAL ABLATION ELECTRODES THAT ARE ASYMMETRIC ELECTRICALLY**

(30) Priority: 18.05.2021 US 202117323909
(71) Applicant: Biosense Webster (Israel) Ltd, Yokneam, 2066717 (IL)
(72) Inventor: GOVARI, Assaf, Yokneam 2066717 (IL); BEECKLER, Christopher Thomas, Irvine 92618 (US)
(74) Representative: Carpmaels & Ransford LLP

(57) **Abstract**

A catheter includes a shaft, a distal-end assembly, and a plurality of electrodes mounted on the distal-end assembly. The shaft is configured for insertion into an organ of a patient. The distal-end assembly is coupled to a distal end of the shaft and configured to make contact with tissue in the organ. At least an electrode among the electrodes is (i) electrically exposed on at least a portion of a surface of the electrode that makes contact with the tissue and (ii) electrically insulated on at least a portion of the surface of the electrode that faces away from the tissue.

## Description

### FIELD OF THE INVENTION

The present invention relates generally to medical probes, and particularly to multi-electrode catheters.

### BACKGROUND OF THE INVENTION

Various known catheter designs have been proposed in the patent literature. For example, U.S. Patent 10,806,511 describes partially insulated focused ablation (PIFA) tip electrode catheters. These catheters use thermally conductive materials that have differential radiofrequency (RF) heating and thereby allow for tailored RF application, to deliver RF energy in a unipolar mode. Open-irrigated, 4 mm, and 8 mm RF ablation catheters were partially insulated by coating half their surfaces with a layer of vinyl, silicone, vinyl-silicone, polyurethane, or a composite of aluminum oxide/boron nitride (AOBN). RF ablation using catheter tips partially coated with a thermally conductive insulation material such as AOBN results in larger ablation lesion volumes without being limited by standard temperature controls. Partial insulation of the catheter tip is able to protect adjacent critical structures during in vivo ablation.

As another example, U.S. Patent Application Publication 2001/0018585 describes relief of urethral obstruction that is achieved by heat ablation of prostatic tissue by an ablation tip-electrode passed within the urethra to a position in the prostate near the point of urethral obstruction. The electrode is coupled to a high frequency power supply to ablatively heat the urethra and the prostatic tissue near the urethra. Image guidance of the electrode placement is monitored by an imaging device. The temperature of the tissue is sensed at the electrode to control the high frequency heating energy and ablation process. The electrode has a blunt tip to help prevent piercing of the wall of the urethra during insertion of the electrode into the urethra through the penis and the positioning of the electrode tip near to the point of urethral obstruction. Several forms of electrodes, apparatus, and methods accommodate the specific objectives.

### SUMMARY OF THE INVENTION

An embodiment of the present invention that is described hereinafter provides a catheter including a shaft, a distal-end assembly, and a plurality of electrodes mounted on the distal-end assembly. The shaft is configured for insertion into an organ of a patient. The distal-end assembly is coupled to a distal end of the shaft and configured to make contact with tissue in the organ. At least an electrode among the electrodes is (i) electrically exposed on at least a portion of a surface of the electrode that makes contact with the tissue and (ii) electrically insulated on at least a portion of the surface of the electrode that faces away from the tissue.

In some embodiments, the electrode is fitted on an arm of the distal-end assembly and is mechanically symmetrical with respect to a 180-degree rotation about a longitudinal axis of the arm.

In some embodiments, the distal-end assembly, when making contact with the tissue, defines an inner volume that is enclosed by the electrodes, wherein the electrode is (i) electrically exposed on at least part of the surface of the electrode that faces out of the inner volume and (ii) electrically insulated on at least part of the surface of the electrode that faces into the inner volume.

In an embodiment, the distal-end assembly is a basket including multiple expandable arms. In another embodiment, the distal-end assembly comprises a helicoid. In some embodiments, the distal-end assembly includes multiple open-ended arms extending from the distal end of the shaft.

In some embodiments, the electrodes are made of flexible printed circuit board (PCB) disposed with the electrical insulation.

There is additionally provided, in accordance with an embodiment of the present invention, a method for manufacturing a catheter, including mounting a plurality of electrodes on a distal-end assembly configured for making contact with tissue in an organ of a patient, wherein at least an electrode among the electrodes is (i) electrically exposed on at least a portion of a surface of the electrode that makes contact with the tissue and (ii) electrically insulated on at least a portion of the surface of the electrode that faces away from the tissue. The distal-end assembly is coupled to a distal end of a shaft. The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based cardiac mapping and ablation system comprising a basket catheter, in accordance with an embodiment of the present invention;
Fig. 2 is a schematic, pictorial illustration of the basket catheter of Fig. 1 fitted with asymmetrically electrically isolated electrodes, in accordance with an embodiment of the present invention;
Figs. 3A and 3B are schematic pictorial illustrations of Lasso^{®} and multi-arm Pentaray^{®} distal-end assemblies, respectively, that are fitted with asymmetrically electrically isolated electrodes, in accordance with embodiments of the present invention; and
Fig. 4 is a flow chart that schematically illustrates a manufacturing method of a multi-electrode catheter fitted with asymmetrically electrically isolated electrodes, in accordance with an embodiment of the present invention.

### DETAILED DESCRIPTION OF EMBODIMENTS

### OVERVIEW

Multi-electrode catheters, such as a basket catheter, are typically fitted with electrodes that can be used to ablate tissue and/or electrophysiologically (EP) map tissue when the electrodes are in contact with the tissue. However, when the ablation and/or EP mapping is being performed, the electrodes are also in contact with the blood pool (e.g., of a cardiac chamber). In case of ablation, the applied electrical energy is also dissipated in the blood. For example, when performing bipolar ablation, such as is typically done in irreversible electroporation (IRE), there could be excessive electrical conduction via blood that may decrease the efficacy of ablation. The blood dissipation is further problematic since it can, for example, generate blood clots.

As another example, when performing bipolar EP mapping, the electrodes may acquire interfering far-field signals via the blood.

In the present context, a bipolar signal is a signal acquired by pairs of electrodes of the catheter. A far-field bio-electric signal is a signal from a region distant from the contacted tissue region. Typically, such a far-field bio-electric signal propagates by conduction through blood.

While blood dissipation and/or far-field propagation may be reduced by constructing the electrodes to have a smaller surface area in contact with the blood, i.e., to have an asymmetry, producing such an asymmetric electrode is expensive.

Some embodiments of the present invention that are described hereinafter provide catheters for insertion into an organ, such as a heart of a patient, which comprise electrodes that are mechanically symmetrical, thus inexpensive to produce.

In an embodiment, a catheter comprises a shaft for insertion into an organ of a patient, and a distal-end assembly, which is coupled to a distal end of the shaft and configured to make contact with tissue in the organ. A plurality of electrodes is mounted on the distal-end assembly, and at least one electrode among the electrodes is (i) electrically exposed on at least a portion of a surface of the electrode that makes contact with the tissue and (ii) electrically insulated on at least a portion of the surface of the electrode that faces away from the tissue. To this end, the electrode is fitted on an arm of the distal-end assembly and is mechanically symmetrical with respect to a 180-degree rotation about a longitudinal axis of the arm.

When making contact with the tissue, the distal-end assembly defines an inner volume that is enclosed by the electrodes, and the electrode is (i) electrically exposed on at least part of the surface of the electrode that faces out of the inner volume and (ii) electrically insulated on at least part of the surface of the electrode that faces into the inner volume.

To form electrodes with the required electrical asymmetry, and taking a basket catheter as an example, an insulator, such as polyurethane, coats the "inner" side of the electrodes, i.e., the side of the electrodes facing away from tissue. In case of a basket catheter, the insulation faces an inner volume defined by the basket. The outer, uncoated side of the electrode is able to deliver electrical energy to contacted tissue.

The disclosed technique is applicable for different catheter geometries, for example, to multi-electrode Lasso^{®} catheters (i.e., comprising a helicoidal distal-end assembly) or Pentaray^{®} catheters (i.e., comprising a multi-ray distal-end assembly in which open-ended arms extend from the distal end of the shaft). The multiple electrodes, that are disposed over one or more arms (e.g., spines) of such catheters, have a geometry that defines, for each electrode, a direction away from tissue, as shown below.

In addition to improving bipolar ablation, the disclosed catheters provide improved EP-sensing capabilities due to the asymmetric electrodes admitting fewer of the far-field electrical signals.

By providing catheters fitted with electrically asymmetric electrodes, cardiac ablation and/or sensing may become more effective.

### SYSTEM DESCRIPTION

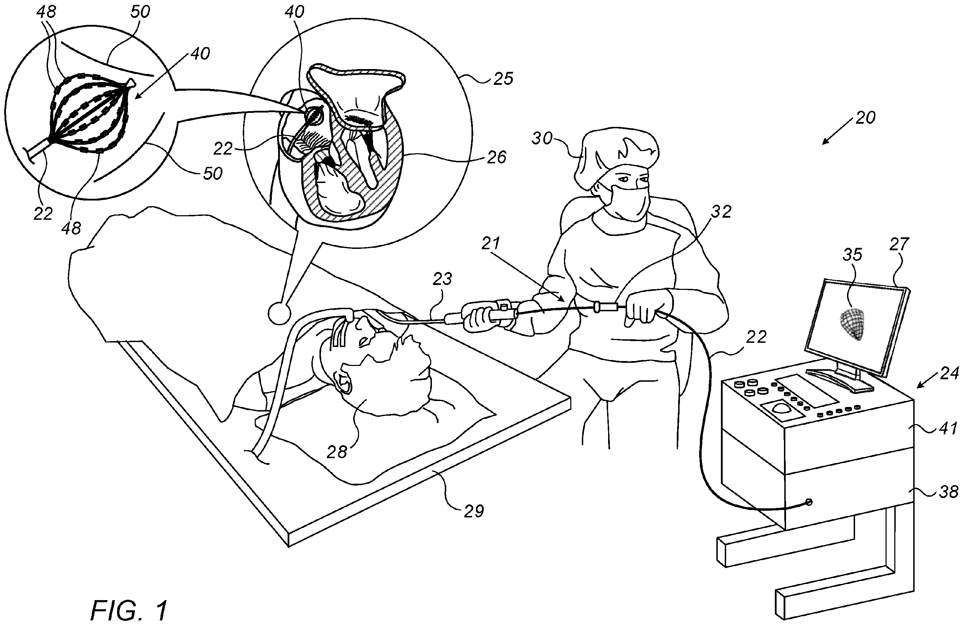

Fig. 1 is a schematic, pictorial illustration of a catheter-based cardiac mapping and ablation system 20 comprising a basket catheter 40, in accordance with an embodiment of the present invention. System 20 comprises a catheter 21, having a shaft 22 that is navigated by a physician 30 into a heart 26 of a patient 28 lying on a table 29. In the pictured example, physician 30 inserts shaft 22 through a sheath 23, while manipulating the distal end of shaft 22 using a manipulator 32 near the proximal end of the catheter and/or deflection from the sheath 23. As shown in an inset 25, basket catheter 40 is fitted at the distal end of shaft 22. Basket catheter 40 comprises multiple expandable arms and is inserted through sheath 23 in a collapsed state and is then expanded within heart 26.

In an embodiment, basket catheter 40 is configured to (i) perform spatial mapping of a cardiac chamber of heart 26 obtaining electrophysiological signals from cardiac chamber surfaces 50, and (ii) apply electrical ablative energy to cardiac chamber surfaces 50. An enlarged view of a portion of inset 25 of Fig. 1 shows basket catheter 40 in inside a cardiac chamber of heart 26. As seen, basket catheter 40 comprises an array of electrodes 48 coupled onto spines that form the basket shape. In one embodiment, the ablation is performed by applying ablative energy between pairs of electrodes 48, in a bipolar ablation mode.

The proximal end of catheter 21 is connected to a console 24. Console 24 comprises a processor 41, typically a general-purpose computer, with suitable front end and interface circuits 38 for transmitting and receiving electrical signals to and from catheter 21, as well as for controlling the other components of system 20. In an embodiment, the surface of the surrounding anatomy is presented to physician 30 on a monitor 27, e.g., in a graphical form of a mesh diagram 35.

Processor 41 is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

Although the pictured embodiment in Fig. 1 relates specifically to the use of a basket catheter for cardiac mapping, other distal-end assemblies may be used, such as the aforementioned helicoidal Lasso^{®} catheter or the multi-arm Pentaray^{®} catheter.

### PHYSICALLY SYMMETRICAL ABLATION ELECTRODES THAT ARE ELECTRICALLY ASYMMETRIC

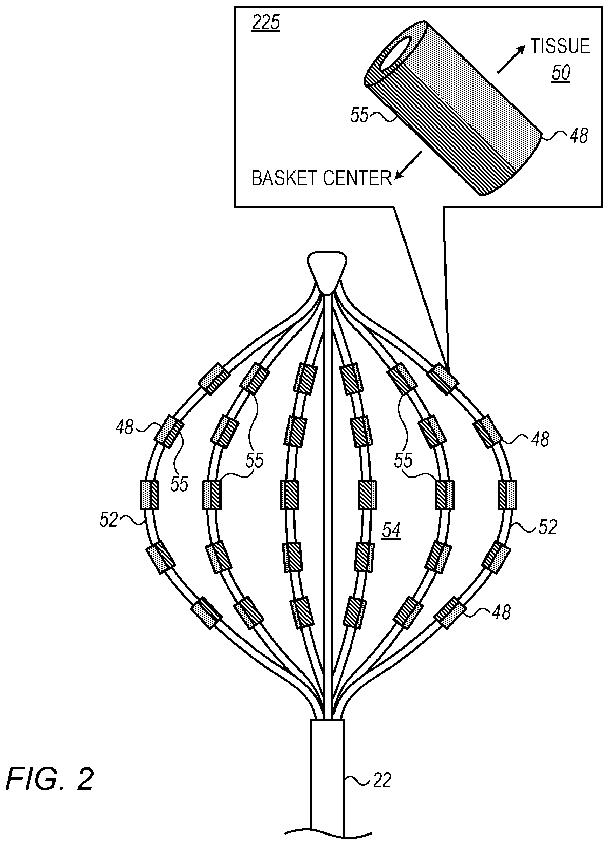

Fig. 2 is a schematic, pictorial illustration of the basket catheter 40 of Fig. 1 fitted with asymmetrically electrically isolated electrodes 48, in accordance with an embodiment of the present invention.

As seen, a distal-end assembly in a form of a basket expandable frame is coupled to a distal end of shaft 22, with the expandable frame comprising a plurality of expandable spines 52 to define an internal lumen 54, such as one defined by a surface of revolution about a longitudinal axis of shaft 22. However, in general, the internal lumen is not required to have rotational symmetry.

In the shown embodiment, at least part of expandable spines 52 is made of flexible printed circuit boards (PCB).

Fig. 2 shows a plurality of electrodes 48 fitted over spines 52. Electrodes 48 are coated on their inner portion with a layer of electrical insulation 55. Inset 225 of Fig. 2 shows an example of an electrode 48, with its metal portion facing tissue 50 while its electrically insulated (55) portion facing blood. Insulation 55 may comprise, for example, polyurethane or any other suitable electrically-insulating material. A longitudinal bore through the electrode, for threading the electrode on a spine 52, is also seen.

In the present example, electrode 48 has a circular (or annular) cross section that has a rotational symmetry about the longitudinal axis of spine 52. Generally, however, full rotational symmetry is not mandatory. In alternative embodiments, electrode 48 may be, for example, symmetrical with respect to a 180-degree rotation about the longitudinal axis of the spine.

When catheter 40 is applied to ablate tissue and/or acquire diagnostic EP signals from tissue, electrodes 48 contact both tissue and blood. However, asymmetric electrical insulation 55 diminishes a portion of applied and/or acquired electrical signals that arrive via blood.

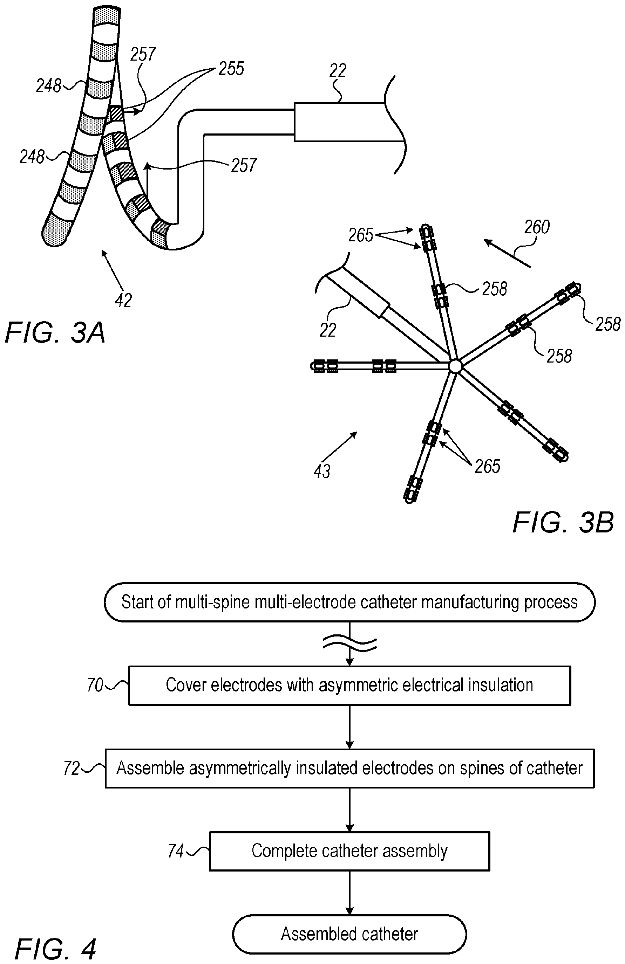

Figs. 3A and 3B are schematic pictorial illustrations of the Lasso^{®} and multi-arm Pentaray^{®} distal-end assemblies 42 and 43, respectively, that are fitted with asymmetrically electrically isolated electrodes, 248 and 258, respectively, in accordance with embodiments of the present invention.

As seen in Fig. 3A, a helicoid distal-end assembly 42 defines a radius of rotation along a longitudinal axis parallel to the distal end of shaft 22, that enable predefining, per electrode 248, an inward radial direction 257 toward the longitudinal axis (as illustrated by arrows 257). As further seen, electrodes 248 are each asymmetrically electrically insulated (255) on a portion of the electrodes along at least the predefined direction 257.

As seen in Fig. 3B, distal-end assembly 43 predefines a proximal direction 260 (as illustrated by arrow 260). As further seen, electrodes 258 are each asymmetrically electrically insulated (265) on a proximal portion of the electrodes along at least the predefined direction 260. Thus, when distal-end assembly 43 presses distally against tissue, its electrodes 258 are mostly in electrical contact with tissue, while their proximal portion is electrically insulated from the blood pool.

### MANUFACTURING METHOD OF CATHETERS WITH MULTIPLE

### ELECTRODES THAT ARE ELECTRICALLY ASYMMETRIC

Fig. 4 is a flow chart that schematically illustrates a manufacturing method of a multi-electrode catheter fitted with asymmetrically electrically isolated electrodes, in accordance with an embodiment of the present invention. In general, manufacturing steps which precede the disclosed manufacturing steps are not shown for simplicity of presentation.

At an asymmetric electrical insulating step 70, electrode parts are processed (e.g., by coating or depositing) to have an electrical insulation layer on a portion of their surfaces, that are shown, by way of example, in Figs. 2 and 3. Next, at an electrode assembly step 72, the electrodes are mounted on the distal end assembly, such as electrode 48 on spines 52 of basket catheter 40. The electrodes are mounted such that the electrically insulated portion faces away from tissue when the catheter engages tissue (i.e., in the aforementioned inner direction for basket catheter 40). At a manufacturing step 74, the catheter is completed (e.g., wired and mechanically locked), to have a ready catheter.

The example flow chart shown in Fig. 4 is chosen purely for the sake of conceptual clarity. In practice, manufacturing stages and manufacturing methods may substantially differ from that presented in this highly simplified flow chart. For example, the electrodes may be made and eclectically insulated by using different materials and processing methods.

Although the embodiments described herein mainly address cardiac catheters, the methods and systems described herein can also be used in other applications, such as in neurology, ophthalmology, and renal denervation.

It will be thus appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A catheter, comprising:
a shaft for insertion into an organ of a patient;
a distal-end assembly, which is coupled to a distal end of the shaft and configured to make contact with tissue in the organ; and
a plurality of electrodes mounted on the distal-end assembly, wherein each of the electrodes is (i) electrically exposed on at least a portion of a surface of the electrode that makes contact with the tissue and (ii) electrically insulated on at least a portion of the surface of the electrode that faces away from the tissue.

2. The catheter according to claim 1, wherein the electrode is fitted on an arm of the distal-end assembly and is mechanically symmetrical with respect to a 180-degree rotation about a longitudinal axis of the arm.

3. The catheter according to claim 1, wherein the distal-end assembly, when making contact with the tissue, defines an inner volume that is enclosed by the electrodes, and wherein each of the electrodes is (i) electrically exposed on at least part of the surface of the electrode that faces out of the inner volume and (ii) electrically insulated on at least part of the surface of the electrode that faces into the inner volume.

4. The catheter according to claim 1, wherein the distal-end assembly is a basket comprising multiple expandable arms.

5. The catheter according to claim 1, wherein the distal-end assembly comprises a helicoidal member.

6. The catheter according to claim 1, wherein the distal-end assembly comprises multiple open-ended arms extending from the distal end of the shaft.

7. The catheter according to claim 1, wherein the electrodes comprises flexible printed circuit board (PCB) disposed with the electrical insulation.

8. A method for manufacturing a catheter, comprising:
mounting a plurality of electrodes on a distal-end assembly configured for making contact with tissue in an organ of a patient, wherein at least an electrode among the electrodes is (i) electrically exposed on at least a portion of a surface of the electrode that makes contact with the tissue and (ii) electrically insulated on at least a portion of the surface of the electrode that faces away from the tissue; and
coupling the distal-end assembly to a distal end of a shaft.

9. The method according to claim 8, wherein the electrode is fitted on an arm of the distal-end assembly and is mechanically symmetrical with respect to a 180-degree rotation about a longitudinal axis of the arm.

10. The method according to claim 8, wherein mounting the electrodes comprises mounting the electrodes on a basket-shaped distal-end assembly comprising multiple expandable arms.

11. The method according to claim 8, wherein mounting the electrodes comprises mounting the electrodes on a helicoid distal-end assembly.

12. The method according to claim 8, wherein mounting the electrodes comprises mounting the electrodes on multiple open-ended arms extending from the distal end of the shaft.

13. The method according to claim 8, wherein the electrodes are made of flexible printed circuit board (PCB) disposed with the electrical insulation.
